# EUROPEAN PATENT SPECIFICATION

(11) **EP 2 679 138 B1**
(45) Date of publication and mention of the grant of the patent: **06.03.2019**
(21) Application number: 12828843.8
(22) Date of filing: 10.04.2012
(51) Int. Cl.: A61B 1/00, G02B 23/24, A61B 1/012

(54) **ENDOSCOPE**
ENDOSKOP
ENDOSCOPE

(30) Priority: 01.09.2011 JP 2011190966
(43) Date of publication of application: 01.01.2014
(73) Proprietor: Olympus Corporation, Tokyo 192-8507 (JP)
(72) Inventor: OTAWARA, Takashi, Hachioji-shi, Tokyo 192-8507 (JP)
(74) Representative: Schicker, Silvia
(86) International application number: PCT/JP2012/059776
(87) International publication number: WO 2013/031280

(56) References cited:
- DE-A1- 3 741 938
- JP-A- H11 178 784
- JP-A- 2000 051 145
- JP-A- 2001 078 955
- JP-A- 2001 137 181
- JP-A- 2002 172 084
- JP-A- 2003 038 421
- JP-A- 2004 121 860
- JP-A- 2006 055 659
- US-A- 5 810 715
- US-A- 5 885 208

## Description

### Technical Field

The present invention relates to an endoscope in which a plurality of rigid tubular members configured to be inserted into a flexible tube portion are disposed in different quadrants.

### Background Art

An endoscope is configured to be able to examine inside a body cavity and observe a target region without performing dissection by inserting an elongated insertion portion into a body cavity, and has an insertion portion configured to be inserted into a body cavity and an operation portion provided on a proximal end side of the insertion portion. Furthermore, the insertion portion includes an elongated flexible tube portion having flexibility, a bending portion configured to perform bending action and provided on a distal end side of the flexible tube portion, and a distal end rigid portion provided on a distal end side of the bending portion. The flexible tube portion is long and has flexibility so as to be insertable even into a flexed insertion path. In addition, the bending portion can be bent in up, down, left and right directions by bending operation in the operation portion.

When the bending portion is bent, the distal end side of the flexible tube portion provided so as to be connected to the bending portion is influenced by the bending of the bending portion, and bent in the same manner as the bending portion. On the other hand, the insertion portion includes inside thereof long tubular members such as an air/water feeding tube, a signal cable, a light guide fiber, etc., in a state where the long tubular members are not bundled and allowed to move in the insertion portion. Allowing the movement of the respective long tubular members in the insertion portion makes it possible to ensure flexibility of the flexible tube portion.

Therefore, when the distal end side of the flexible tube portion is bent due to the bending of the bending portion, a radius of curvature of bending outer periphery becomes large and a radius of curvature of bending inner periphery becomes small. As a result, a tubular member disposed on a bending outer peripheral side is pulled, which causes the tubular member to attempt to move in a bending inner peripheral direction. On the other hand, a tubular member disposed on a bending inner peripheral side is compressed, which creates slackness and causes the part where the slackness is created to attempt to move in the bending outer peripheral direction.

In a case where such an endoscope is a side-view endoscope as disclosed in Japanese Patent Application Laid-Open Publication No. 2003-38421, for example, the side-view endoscope includes at the distal end portion thereof a raising stand configured to raise a distal end portion of a treatment instrument. The raising stand and a treatment instrument raising operation portion provided on an operation portion side are connected to each other through a raising operation wire, and the raising stand is raised by operation of the treatment instrument raising operation portion through the raising operation wire. The raising operation wire is advanceably/retractably inserted into a wire guide coil which is inserted in the insertion portion. Since the wire guide coil is rigid, if subjected to a tensile load or a compression load in an axial direction in association with the bending action of the bending portion, the wire guide coil pushes away other soft tubular members in an attempt to move in the bending inner peripheral direction or in the bending outer peripheral direction. Further, an operation wire tube and a light guide tube are arranged in the same quadrant.

In addition, as recited in the above-described document, it is well-known to provide a side-view endoscope with a rigidity adjusting mechanism which enables a rigidity of a flexible tube portion to be arbitrarily adjusted. The rigidity adjusting mechanism is configured to adjust the rigidity of the flexible tube portion by operating the rigidity adjusting operation portion provided in the operation portion, and the flexible tube portion includes inside thereof: a rigidity adjusting wire having one end connected to the rigidity adjusting operation portion and the other end fixed to the distal end of the flexible tube portion; and a rigidity adjusting coil provided outwardly on the rigidity adjusting wire. An operator compresses the rigidity adjusting coil by pulling the rigidity adjusting wire through the rigidity adjusting operation portion, and adjusts the flexible tube portion to a desired rigidity according to the density of the coil
at that time.

Incidentally, the above-described rigidity adjusting coil is also rigid. Therefore, if the rigidity adjusting coil is disposed on the bending outer periphery, a tensile load acts in the axial direction in accordance with the bending action of the bending portion, which causes the rigidity adjusting coil to attempt to move in the bending inner peripheral direction, and if the rigidity adjusting coil is disposed on the bending inner periphery, a compression load acts in the axial direction, which causes the portion where slackness is created to attempt to move in the bending outer peripheral direction.

As a result, in a case where at least two long rigid tubular members (the wire guide coil and the rigidity adjusting coil in the above-described document) are disposed in a flexible tube portion, if the two long rigid tubular members move in the same direction in accordance with the bending operation of the bending portion, there is a possibility that other soft tubular members are not pushed aside by the rigid tubular members, in other words, the soft tubular members cannot get out of the way of the rigid tubular members, and resultantly the soft tubular members get squished.

In view of the above-described circumstances, an object of the present invention is to provide an endoscope which includes at least two rigid tubular members in a flexible tube portion, and which is configured such that a soft tubular member is hard to be squished even if the respective rigid tubular members move in a bending outer peripheral direction or in a bending inner peripheral direction by a bending operation of a bending portion and capable of improving a durability of the soft tubular member.

### Disclosure of Invention

### Means for Solving the Problem

An endoscope according to the present invention comprises the features according to claim 1.

### Brief Description of the Drawings

FIG. 1 is a schematic configuration diagram of a side-view endoscope.
FIG. 2 is a cross-sectional side view of a main part of an insertion portion of the side-view endoscope.
FIG. 3 is a cross-sectional view taken along the line III-III of FIG. 2.
FIG. 4 is a cross-sectional view taken along the line IV-IV of FIG. 2.
FIG. 5 is an illustration diagram of quadrants formed by sectionalizing the cross section of a flexible tube portion with two coordinate axes perpendicular to each other.
FIG. 6 is a cross-sectional view taken along the line VI-VI of FIG. 2.
FIG. 7 is a cross-sectional view corresponding to FIG. 6 during bending action.

### Best Mode for Carrying Out the Invention

Hereinafter, an embodiment of the present invention will be described with reference to drawings. Note that each of the drawings is a pattern diagram, and care should be taken to the fact that the relationship between the thicknesses and the widths of the respective members, a ratio of the thickness of a certain member to that of another member, and the like are different from those in the actual members. It is needless to say that each of the drawings includes a part including a different relationship and a different ratio among the dimensions of the respective members.

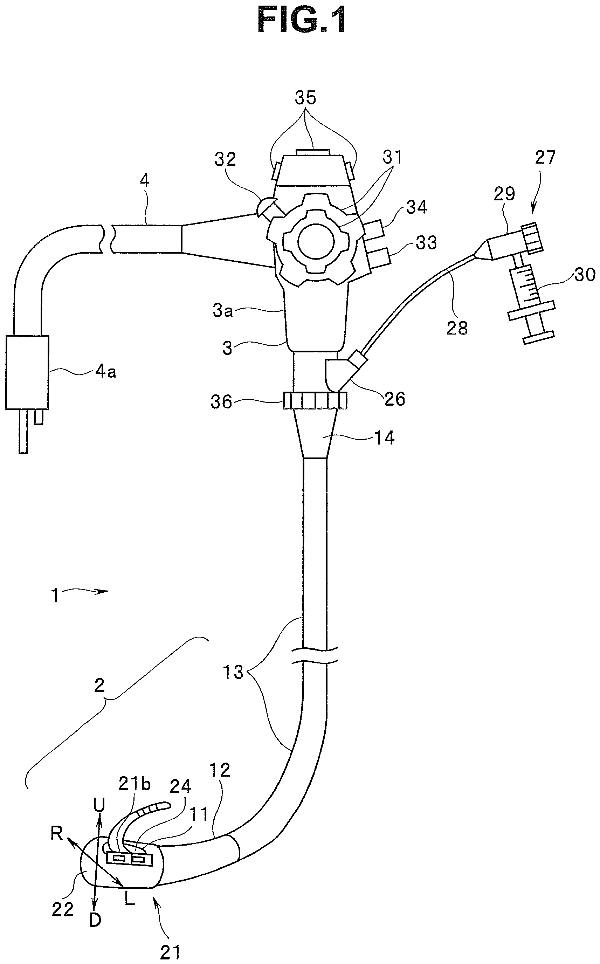

The reference numeral 1 in FIG. 1 represents a side-view endoscope as one example of an endoscope, and the endoscope 1 is adopted as a duodenum endoscope, for example.

The endoscope 1 includes an elongated insertion portion 2, a wide-width operation portion 3 provided in a linked manner on a rear end side of the insertion portion 2, and a universal cord 4 extended from a side portion of the operation portion 3. At an end portion of the universal cord 4, a connector portion 4a to be connected to a light source apparatus and a camera control unit, not shown, is provided.

The insertion portion 2 includes, in the following order from a distal end side, a rigid distal end portion 11, a bending portion 12 provided in a linked manner at a rear end of the distal end portion 11, and a long flexible tube portion 13 which has flexibility and provided in a linked manner at a rear end of the bending portion 12. In addition, a break prevention member 14 formed in a tapered shape is provided on an outer periphery of the rear end of the flexible tube portion 13, and the rear end of the flexible tube portion 13 and a front end of the operation portion 3 are connected to each other through the break prevention member 14.

The distal end portion 11 includes a main body portion 21 having an observation window, an illumination window, and the like, not shown, and a distal end cover member 22 for covering and electrically insulating the main body portion 21. In addition, a housing space 24 for housing a treatment instrument raising stand 23 is provided at a side portion of the main body portion 21. On the other hand, the operation portion 3 includes a grasping portion 3a and a treatment instrument insertion port 26 is open at the grasping portion 3a.

The operation portion 3 is provided with: two bending operation knobs 31 for causing the bending portion 12 to bend in an up/down (U-D) direction and a left/right (L-R) direction as bending directions of the bending portion; a treatment instrument raising lever 32 for remotely operating the raising operation of the treatment instrument raising stand 23; an air/water feeding operation button 33; a suction operation button 34; a control switch operation button 35; and the like. Furthermore, a rigidity adjusting ring 36 which adjusts the rigidity of the flexible tube portion 13 is provided at the front end portion of the operation portion 3 which is adjacent to the break prevention member 14. The rigidity adjusting ring 36 is connected to a rigidity adjusting mechanism 57, to be described later, which is inserted in the flexible tube portion 13, and the rigidity adjusting mechanism 57 varies the rigidity of the flexible tube portion 13 by the rigidity adjusting ring 36 being rotated, which facilitates the insertion into a flexed insertion path.

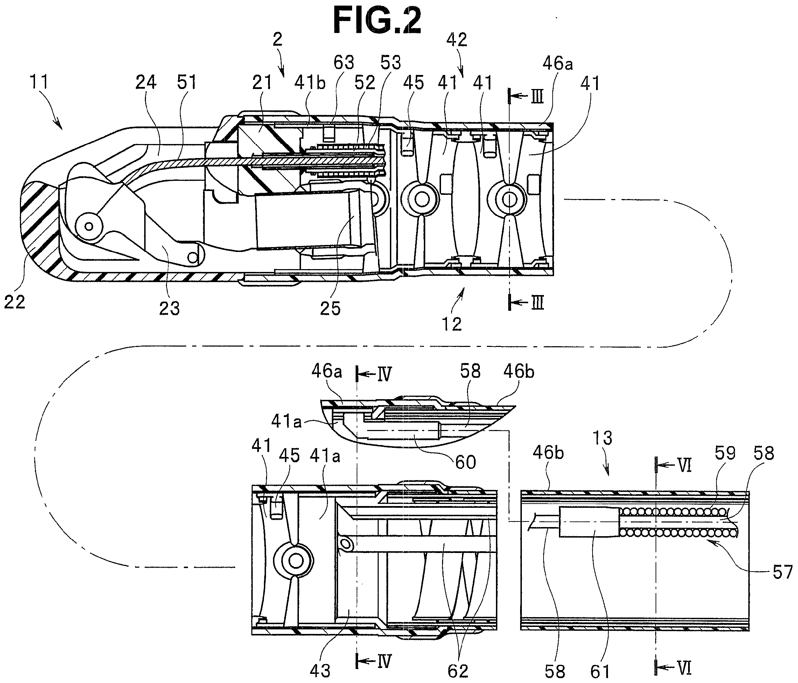

As shown in FIG. 2, the bending portion 12 includes a bending piece group 42 including a plurality of bending pieces (joint rings) 41 formed in a circular ring shape such that bending pieces 41 adjacent to each other are rotatably connected to each other with the phases of the adjacent bending pieces being changed by 90 degrees with respect to each other, and the adjacent bending pieces 41 are rotated relatively with respect to each other, thereby allowing the bending piece group 42, as a whole, to bend in the up/down direction and the left/right direction. A rear portion of the bending piece group 42 is fixed to a front ferrule 43 which is fixed to the distal end of the flexible tube portion 13. Note that the reference numerals 46a, 46b represent outer tubes for covering the outer peripheries of the bending portion 12 and the flexible tube portion 13.

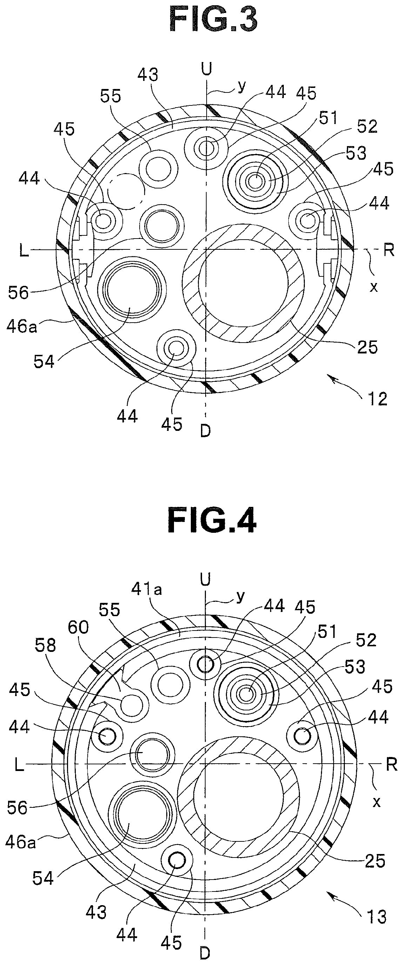

In addition, coil pipes 62 configured to guide four bending operation wires 44 which cause the bending portion 12 to bend are disposed in the insertion portion 2, and the distal ends of the coil pipes 62 are fixed to the front ferrule 43. Furthermore, supporting rings 45 through which the respective bending operation wires 44 are inserted and which support the bending operation wires are fixed to the respective bending pieces 41. The respective supporting rings 45 and the respective coil pipes 62 are disposed respectively at positions close to an inner wall surface of the bending portion 12 in the up, down, left and right (U, D, L, R) bending directions. The respective bending operation wires 44 are inserted into the corresponding coil pipes 62 and supporting rings 45, respectively, and the distal ends of the bending operation wires are connected to a wire fixing portion 63 fixed to the front-most bending piece 41b. Note that, for simplification of the drawing, the tubular members located at the portion other than the main portion are omitted in FIG. 2. However, actually, various kinds of tubular members are inserted in a predetermined manner, as shown in FIGS. 3, 4 and 6 to be described later.

In addition, the proximal end sides of the bending operation wires 44 are extended to the side of the operation portion 3 through inside of the insertion portion 2, and connected to the two bending operation knobs 31, respectively, through a pulling mechanism for bending operation (not shown). When an operator operates the bending operation knobs 31 to pull any one of the bending operation wires 44, the bending portion 12 is bent in the direction of the pulled bending operation wire 44.

In addition, as shown in FIGS. 3 and 4, various kinds of tubular members including a treatment instrument channel 25 are inserted in the insertion portion 2. The treatment instrument channel 25 is made of a flexible channel tube, and has a distal end which opens in the housing space 24 and a rear end which communicates with the treatment instrument insertion port 26. A treatment instrument 27 is inserted from the treatment instrument insertion port 26. The treatment instrument 27 passes through inside of the treatment instrument channel 25 and the distal end portion of the treatment instrument is guided to the housing space 24. Note that a contrast tube is shown in the drawings as one example of the treatment instrument 27. In the drawings, the reference numeral 28 represents a flexible tube, 29 represents a syringe connector, and 30 represents a syringe for contrast agent injection.

The distal end of the treatment instrument raising wire 51 is connected to the treatment instrument raising stand 23, and the rear end of the treatment instrument raising wire 51 is connected to the treatment instrument raising lever 32. The treatment instrument raising stand 23 can be raised by operating the treatment instrument raising lever 32. The treatment instrument raising wire 51 is inserted through the flexible tube portion 13 and the bending portion 12 while being advanceably/retractably inserted in a guide tube 52, and furthermore, a wire guide coil 53 as a rigid tubular member is inserted over the outer periphery of the guide tube 52. The distal end of the wire guide coil 53 is fixed to the main body portion 21 provided to the distal end portion 11, and the rear end of the wire guide coil is disposed on the side of the treatment instrument insertion port 26. In addition, the reference numeral 54 represents a light guide fiber bundle, 55 represents an air/water feeding tube, and 56 represents a signal cable connected to an image pickup apparatus, not shown.

Furthermore, the rigidity adjusting mechanism 57 is disposed in the flexible tube portion 13. As shown in FIGS. 2 and 4, the rigidity adjusting mechanism 57 includes a rigidity adjusting wire 58 and a rigidity adjusting coil 59 arranged outwardly on the rigid adjusting wire 58. The rigidity adjusting coil 59 is configured as a second tubular member (hereinafter, also referred to as "rigid tubular member") which is more rigid than the air/water feeding tube 55, a signal cable 56, and the like which are configured as first tubular members (hereinafter, also referred to as "soft tubular members"). The distal end of the rigidity adjusting wire 58 is fixed to the rear-most bending piece 41a which is fixed to the front ferrule 43 through a connecting member 60, and the rear end of the rigidity adjusting wire is connected to the rigidity adjusting ring 36 through a pulling member (not shown). Note that, in FIG. 2, the connecting member 60 is shown with a cross section of a portion different from the portion where the coil pipe 62 is shown.

A stopper member 61 is fixed to the distal end side of the rigidity adjusting wire 58, and the distal end of the rigidity adjusting coil 59 is latched on the stopper member 61. Furthermore, the rear end of the rigidity adjusting coil 59 is latched on the side of the pulling member (not shown). When the rigidity adjusting ring 36 is rotated in a fastening direction, the rigidity adjusting wire 58 is pulled through the pulling member, and thereby the stopper member 61 compresses the rigidity adjusting coil 59 toward the side of the operation portion 3. As a result, the rigidity of the flexible tube portion 13 is adjusted according to the density of the rigidity adjusting coil 59. Note that the above-described respective tubular members are inserted in the flexible tube portion 13 without being bundled, and allowed to move inside the flexible tube portion 13 when the flexible tube portion is flexed.

Incidentally, the treatment instrument raising wire 51 and the rigidity adjusting wire 58 are inserted in the flexible tube portion 13 with the movement in a diameter direction being allowed, thereby ensuring the flexibility of the flexible tube portion 13. Therefore, when the bending portion 12 is bent, in a case where the respective wires 51, 58 are disposed on the bending outer peripheral side, a tensile load acts, which causes the respective wires to move in the bending inner peripheral direction in an attempt to take the shortest route. On the other hand, in a case where the wires are disposed on the bending inner peripheral side, slackness is created by a compression load acting from the axial direction, which causes the part where the slackness is created to attempt to move in the bending outer peripheral direction.

The coils 53, 59 through which the respective wires 51, 58 are inserted are metal coils, and rigid and elastic, so that these coils are not crushed even if the coils are subjected to an external pressure. However, if the respective coils 53, 59 compress other soft tubular members (air/water feeding tube 55, the signal cable 56, etc.) and the light guide fiber bundle 54 as a solid soft tubular member, the soft tubular members are likely to be crushed because these members are not elastic, and weak and brittle to an external pressure. Similarly, the signal cable 56 is weaker than the coils 53, 59, and is easily to be crushed when subjected to an external pressure. Note that the respective bending operation wires 44 are also rigid. However, the bending operation wires are supported by being inserted through the coil pipes 62 and the supporting rings 45. Therefore, the bending operation wires do not move in the bending outer peripheral direction or in the bending inner peripheral direction, even when the bending portion 12 is bent.

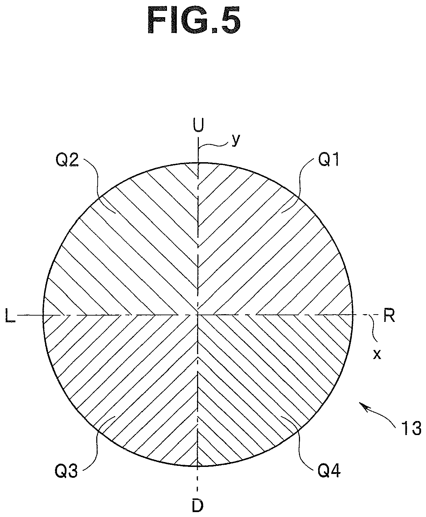

Therefore, in the present embodiment, as shown in FIG. 5, the cross section of the flexible tube portion is sectionalized into a first to fourth quadrants Q1 to Q4 with coordinate axes (hereinafter, referred to as "x-axis" and "y-axis") passing through two rotational centers, which are perpendicular to each other, of the respective bending pieces 41 configuring the bending piece group 42 of the bending portion 12. Then, the respective quadrants Q1 to Q4 are made to correspond to the flexible tube portion 13 in a state where the insertion portion 2 is stretched in a straight line, and the centers of the axes of the respective rigid tubular members 53, 59, and 25 inserted in the flexible tube portion 13 are disposed so as to be dispersed in the respective quadrants Q1 to Q4. Note that, in this case, the x-axis is the rotational center of the up/down (U-D) direction, and the y-axis is the rotational center of the left/right (L-R) direction.

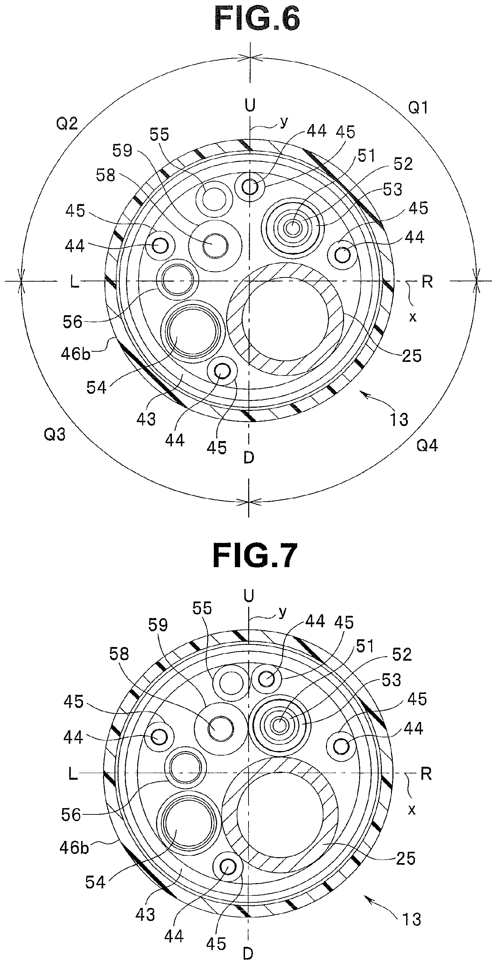

Specifically, as shown in FIG. 6, in a state where the bending operation wires 44 and the rigidity adjusting wire 58 are relaxed, the center of the axis of the wire guide coil 53 having the treatment instrument raising wire 51 inserted therethrough is disposed in the first quadrant Q1 (R-U region), the center of the axis of the rigidity adjusting coil 59 having the rigidity adjusting wire 58 inserted therethrough is disposed in the second quadrant Q2 (U-L region), and furthermore, the center of the axis of the treatment instrument channel 25 is disposed in the fourth quadrant (D-R region). Note that, in the third quadrant (L-D region), the center of the axis of the light guide fiber bundle 54 having the lowest rigidity among the soft tubular members is disposed.

In such a configuration, when the operator operates the bending operation knobs 31 to bend the bending portion 12 in the left (L) direction, the wire guide coil 53 disposed on the bending outer side moves in the bending inner peripheral direction in an attempt to take the shortest route from inside of the flexible tube portion 13 to the bending portion 12, due to a tensile load acting in the distal end direction by the bending action of the bending portion 12. In addition, since a compression load acts from the front ferrule 43 side to rearward in the axial direction of the rigidity adjusting coil 59 disposed on the bending inner peripheral side, which creates slackness at the halfway part of the coil. In this case, the rigidity adjusting coil 59 is rigid, so that the part where slackness is created bows and moves in the bending outer peripheral direction.

On the other hand, when the bending portion 12 is bent in the right (R) direction, the rigidity adjusting coil 59 disposed on the bending outer side moves in the bending inner peripheral direction in an attempt to take the shortest route from inside of the flexible tube portion 13 to the bending portion 12 due to the tensile load generated in the direction of the distal end portion fixed to the rear-most bending piece 41a by the influence of the bending action of the bending portion 12. In addition, a compression load acts from the front end side on the wire guide coil 53 disposed on the bending inner peripheral side, thereby creating slackness at the halfway part of the wire guide coil and causing the slack part to bend and move in the bending outer peripheral direction.

Both of the coils 53 and 59 are rigid and elastic as well. Therefore, if slackness is generated at the halfway parts of the coils, the slack parts largely flex in the bending outer peripheral direction. Then, as shown in FIG. 7, in accordance with the bending action of the bending portion 12 in the left/right direction, both of the rigid coils 53, 59 move in the y-axis direction, press each other, and the movement of the coils is restricted at a balanced position. As a result, in the vicinity of the front ferrule 43 which is on the distal end side of the flexible tube portion 13, the soft tubular members (the tubes 54, 55, the signal cable 56, etc.) are not compressed by the rigid coils 53, 59, thereby preventing damage on the soft tubular members. In addition, even if the bending portion 12 is bent in the left/right direction, the soft tubular members will not be damaged, which greatly increases the durability of the soft tubular members.

Furthermore, in the present embodiment, the treatment instrument channel 25 is disposed in the fourth quadrant Q4 which is different from the quadrants Q1, Q2 where both of the rigid coils 53, 59 are disposed, respectively. Therefore, even if the bending portion 12 is bent in the left/right direction, the treatment instrument channel 25 moves in the left/right direction in FIG. 6, and does not press the wire guide coil 53 disposed in the first quadrant Q1. In addition, since the light guide fiber bundle 54 disposed in the third quadrant Q3 only moves in a substantially horizontal direction in FIG. 7, the light guide fiber bundle does not further press one of or both of the coils 53 and 59.

As a result, even if the bending portion 12 is bent in the left/right direction, one of the soft tubular members is not pressed concurrently by both of the rigid coils 53, 59 and the treatment instrument channel 25, and for example, the light guide fiber bundle 54 is pressed only by the treatment instrument channel 25. That is, the light guide fiber bundle is compressed with a weak pressing force, thereby capable of preventing damage on the light guide fiber bundle 54.

Similarly, if the bending portion 12 is bent in the up/down direction, the wire guide coil 53 disposed in the first quadrant Q1 and the treatment instrument channel 25 disposed in the fourth quadrant Q4 press each other, thereby restricting further movement thereof. At that time, the rigidity adjusting coil 59 moves in the x-axis direction substantially in parallel with the wire guide coil 53, thereby preventing the one soft tubular member (for example, the light guide fiber bundle 54) from being concentrically pressed by two or more rigid tubular members (coils 53, 59, treatment instrument channel 25). Therefore, the soft tubular member (for example, the light guide fiber bundle 54) disposed in the third quadrant Q3 is compressed with a weak pressing force, thereby forestalling damage on the soft tubular member.

Furthermore, when the bending portion 12 is bent in the left/right direction, the position of the wire guide coil 53 is restricted by the rigidity adjusting coil 59 in the vicinity of the front ferrule 43, which prevents the wire guide coil from moving needlessly in the y-axis direction in the bending portion 12. In addition, as shown in FIG. 3, inside the bending portion 12, a vacant space is secured at a region (position shown by one-dot chain line in the drawing) positioned on an extension of the distal end of the rigidity adjusting coil 59.

As a result, even if the wire guide coil 53 moves in the y-axis direction in accordance with the bending action of the bending portion 12, the moving amount of the wire guide coil is restricted. In addition, the vacant space for allowing the soft tubular members to escape is ensured, thereby effectively protecting the soft tubular members against damage.

Thus, in the present embodiment, the three kinds of rigid tubular members (the respective coils 53 and 59, and the treatment instrument channel 25) which are likely to press the soft tubular members are disposed in the different quadrants Q1, Q2 and Q4, respectively. Therefore, even if the bending portion 12 is bent, one of the soft tubular members is not concurrently compressed by two rigid tubular members, and the pressing force is dispersed. As a result, it is possible to effectively protect the soft tubular members against damage.

Note that the present invention is not limited to the above-described embodiments. That is, it is needless to say that the present invention is not limited to the case where the bending portion 12 is bent individually in up, down, left and right directions, for example, but can work similarly in the bending operation in all directions including a twisting state in which the bending operation in up direction or down direction and the bending operation in left direction or right direction are performed simultaneously, and in a bending state of the flexible tube portion 13 itself.

## Claims

1. An endoscope (1) comprising:
an insertion portion (2) including a bending portion (12) including a plurality of bending pieces (41) formed in a circular ring shape such that bending pieces (41) adjacent to each other are rotatably connected to each other with the phases of the adjacent bending pieces being changed by 90 degrees with respect to each other to bend in a first direction (U-D) and a second direction (L-R) orthogonal to the first direction; said insertion portion (2) further including a flexible tube portion (13) provided at a rear end of the bending portion (12) in a linked manner;
a light guide fiber bundle (54) inserted in the insertion portion (2); and
a guide coil (53) through which a raising wire (51) configured to transmit a rotation raising operation of a raising stand (23) for raising a distal end portion of a treatment instrument (27) is inserted, a rigidity adjusting coil (59) configured to adjust a rigidity of the flexible tube portion (13), and a treatment instrument channel (25), the guide coil (53), the rigidity adjusting coil (59) and the treatment instrument channel (25) being inserted in the insertion portion (2) and formed to be more rigid than the light guide fiber bundle (54), wherein
the flexible tube portion (13) is sectionalized into four quadrants (Q1-Q4) by coordinate axes (x, y) passing through two respective rotational centers of respective bending pieces (41) at the time that the bending portion (12) is bent in the first direction (U-D) and in the second direction (L-R), the four quadrants (Q1-Q4) being a first quadrant (Q1), a second quadrant (Q2) adjacent to the first quadrant (Q1), a third quadrant (Q3) adjacent to the second quadrant (Q2), and a fourth quadrant (Q4) adjacent to the third quadrant (Q3) and the first quadrant (Q1),
**characterized in that** a center of the axis of the guide coil (53) is disposed in the first quadrant (Q1), a center of the axis of the rigidity adjusting coil (59) is disposed in the second quadrant (Q2), a center of the axis of the light guide fiber bundle (54) is disposed in the third quadrant (Q3), and a center of the axis of the treatment instrument channel (25) is disposed in the fourth quadrant (Q4).

## Patentansprüche

1. Endoskop (1), umfassend:
einen Einführungsabschnitt (2) mit einem Biegeabschnitt (12), der eine Mehrzahl von Biegeteilen (41) umfasst, die in einer kreisringförmigen Form so ausgebildet sind, dass die zueinander benachbarten Biegeteile (41) drehbar miteinander verbunden sind, wobei die Phasen der benachbarten Biegeteile um 90 Grad zueinander geändert werden, um sich in einer ersten Richtung (U-D) und einer zu der ersten Richtung orthogonalen zweiten Richtung (L-R) zu biegen, wobei der Einführungsabschnitt (2) ferner einen flexiblen Schlauchabschnitt (13) umfasst, der an einem hinteren Ende des Biegeabschnitts (12) angekoppelt vorgesehen ist;
ein Lichtleiterfaserbündel (54), das in den Einführungsabschnitt (2) eingeführt ist; und
eine Führungsspule (53), durch die ein Hubdraht (51), der dazu konfiguriert ist, einen Drehhebevorgang eines Hubgestells (23) zum Anheben eines distalen Endabschnitts eines Behandlungsinstruments (27) zu übertragen, eingeführt wird, eine Steifigkeitseinstellspule (59), die dazu konfiguriert ist, eine Steifigkeit des flexiblen Schlauchabschnitts (13) einzustellen, und einen Behandlungsinstrumentenkanal (25), wobei die Führungsspule (53), die Steifigkeitseinstellspule (59) und der Behandlungsinstrumentenkanal (25) in den Einführungsabschnitt (2) eingeführt und so ausgebildet sind, dass sie steifer sind als das Lichtleitfaserbündel (54), wobei
der flexible Schlauchabschnitt (13) in vier Quadranten (QI-Q4) durch Koordinatenachsen (x, y) unterteilt ist, die durch zwei jeweilige Drehzentren von jeweiligen Biegeteilen (41) zu dem Zeitpunkt hindurchlaufen, zu dem der Biegeabschnitt (12) in der ersten Richtung (U-D) und in der zweiten Richtung (L-R) gebogen ist, wobei die vier Quadranten (Q1-Q4) ein erster Quadrant (Q1), ein zum ersten Quadranten (Q1) benachbarter zweiter Quadrant (Q2), ein zum zweiten Quadranten (Q2) benachbarter dritter Quadrant (Q3) und ein zum dritten Quadranten (Q3) und dem ersten Quadranten (Q1) benachbarter vierter Quadrant (Q4) sind,
**dadurch gekennzeichnet, dass**
eine Mitte der Achse der Führungsspule (53) in dem ersten Quadranten (Q1) angeordnet ist, eine Mitte der Achse der Steifigkeitseinstellspule (59) in dem zweiten Quadranten (Q2) angeordnet ist, eine Mitte der Achse des Lichtleiterfaserbündels (54) im dritten Quadranten (Q3) angeordnet ist und eine Mitte der Achse des Behandlungsinstrumentenkanals (25) im vierten Quadranten (Q4) angeordnet ist.

## Revendications

1. Endoscope (1) comprenant :
une partie d'insertion (2) comprenant une partie de courbure (12) comprenant une pluralité de pièces de courbure (41) formées suivant une forme d'anneau circulaire de telle sorte que des pièces de courbure (41) adjacentes l'une à l'autre sont reliées de manière rotative l'une à l'autre avec les phases des pièces de courbure adjacentes qui sont changées de 90 degrés l'une par rapport à l'autre pour se courber dans une première direction (U-D) et dans une seconde direction (L-R) orthogonale à la première direction ; ladite partie d'insertion (2) comprenant en outre une partie tube souple (13) prévue à une extrémité arrière de la partie de courbure (12) d'une manière reliée ;
un faisceau de fibres de guidage de lumière (54) inséré dans la partie d'insertion (2) ; et
une bobine de guidage (53) à travers laquelle est inséré un fil de levage (51) configuré pour transmettre une opération de levage en rotation d'un socle de levage (23) pour élever une partie d'extrémité distale d'un instrument de traitement (27), une bobine de réglage de rigidité (59) configurée pour régler une rigidité de la partie tube souple (13), et un canal d'instrument de traitement (25), la bobine de guidage (53), la bobine de réglage de rigidité (59) et le canal d'instrument de traitement (25) étant insérés dans la partie d'insertion (2) et formés pour être plus rigides que le faisceau de fibres de guidage de lumière (54),
la partie tube souple (13) étant divisée en quatre quadrants (Q1-Q4) par des axes de coordonnées (x,y) passant par deux centres de rotation respectifs de pièces de courbure respectives (41) au moment où la partie de courbure (12) est courbée dans la première direction (U-D) et dans la seconde direction (L-R), les quatre quadrants (Q1-Q4) étant un premier quadrant (Q1), un deuxième quadrant (Q2) adjacent au premier quadrant (Q1), un troisième quadrant (Q3) adjacent au deuxième quadrant (Q2), et un quatrième quadrant (Q4) adjacent au troisième quadrant (Q3) et au premier quadrant (Q1),
**caractérisé par le fait que**
un centre de l'axe de la bobine de guidage (53) est disposé dans le premier quadrant (Q1),
un centre de l'axe de la bobine de réglage de rigidité (59) est disposé dans le deuxième quadrant (Q2),
un centre de l'axe du faisceau de fibres de guidage de lumière (54) est disposé dans le troisième quadrant (Q3), et
un centre de l'axe du canal d'instrument de traitement (25) est disposé dans le quatrième quadrant (Q4).
